(19) Europäisches Patentamt
European Patent Office
Office européen des brevets

(11) Publication number: **0 048 107**
**A2**

(12) **EUROPEAN PATENT APPLICATION**

(21) Application number: 81303970.8

(22) Date of filing: 01.09.81

(51) Int. Cl.³: C 07 D 307/935
A 61 K 31/34

(30) Priority: 15.09.80 US 187284

(43) Date of publication of application: 24.03.82 Bulletin 82/12

(84) Designated Contracting States: BE CH DE FR GB IT LI NL SE

(71) Applicant: THE UPJOHN COMPANY
301 Henrietta Street
Kalamazoo, Michigan 49001(US)

(72) Inventor: Tiffany, Burris Dwight
6624 Westchester
Kalamazoo Michigan(US)

(74) Representative: Perry, Robert Edward et al,
GILL JENNINGS & EVERY 53-64 Chancery Lane
London WC2A 1HN(GB)

(54) **Crystalline prostaglandins.**

(57) Substantially pure, crystalline forms of the methyl ester and sodium salt of prostaglandin $I_2$.

EP 0 048 107 A2

CRYSTALLINE PROSTAGLANDINS

The present invention relates to crystalline forms, which can be substantially pure, of prostaglandin derivatives.

The prostaglandins and their derivatives are well known organic compounds derived from prostanoic acid, which are useful for a variety of pharmacological purposes. See for example, Bergstrom et al., Pharmacol. Rev. 20:1 (1968), and U.S. Patent Specification No. 4,158,667.

A trivial system of nomenclature has been devised which classifies prostaglandin derivatives based on the substituents around the cyclopentane ring. See, for example N.A. Nelson, J. Med. Chem. 17:911-918 (1974). This system of nomenclature has been extended to compounds such as that of the formula

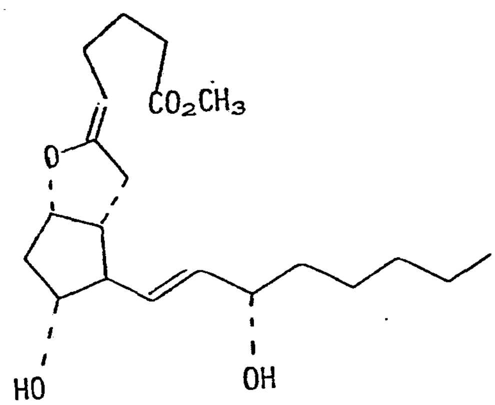

which is one of a class of prostaglandin derivatives known as the prostacyclins. The compound of the given formula may be named 9-deoxy-6,9-epoxy-$PGF_{2\alpha}$, methyl ester, or prostaglandin $I_2$, methyl ester. For a further discussion of the prostacyclins, see U.S. Patent Specificiation No. 4,158,667 and Pace-Asciak et al., Biochem. 10:3657 (1971). Such compounds will be named herein under the PGI nomencalture.

In the given formula, the broken lines of attachment to the solid cyclopentane ring and to the side-chain indicate substituents in the alpha configuration, i.e. below the plane of the ring. Heavy solid line attachments indicate substituents in beta configuration, i.e. above the plane of the ring.

A method for preparing crystals of prostaglandin

$I_2$, methyl ester is disclosed in U.S. Patent Specification No. 4,158,667. Johnson et al., J.A.C.S. 100:24 (1978), disclose that the said method produces prostaglandin $I_2$, methyl ester as soft crystals having a melting point of 30-33°C. The sodium salt of prostaglanding $I_2$, methyl ester is disclosed in Derwent Farmdoc Nos. 5711Y/33, 81213Y/46 and 29115A/16, German Offenlegungsschrift 2720999, and Johnson et al., Prostaglandins 12:915 (1976).

A first novel product of the present invention is a substantially pure crystalline form of prostaglandin $I_2$, methyl ester, characterised in that it exhibits a melting point of from 54.7 to 60.6°C; has $[\alpha]_D$ in ethanol of 96-104° (C = 1.0333); and exhibits IR peaks (in a mineral oil mull) essentially at 3451, 3393, 2922, 2855, 2724, 2682, 1794, 1744, 1711, 1697, 1687, 1457, 1450, 1437, 1417, 1377, 1361, 1352, 1333, 1316, 1286, 1282, 1265, 1240, 1228, 1206, 1175, 1163, 1133, 1127, 1108, 1084, 1069, 1052, 1041, 1022, 1008, 988, 973, 955, 910, 892, 857, 833, 827, 814, 781, 755, 727, 721, 707, 663 and 613 $cm^{-1}$.

A scond novel product of the present invention is a free-flowing, substantially pure form of prostaglandin $I_2$ sodium salt characterised in being greater than 99.0 percent pure by weight, using HPLC analysis.

A pharmaceutical composition according to the invention comprises, in addition to an inert carrier, a first or second product as defined above, or prostaglandin $I_2$, sodium salt, which is greater than 99.0 percent pure by weight, using HPLC analysis, obtained from prostaglandin $I_2$, methyl ester.

The novel pure form of prostaglandin $I_2$, methyl ester is useful for the same purposes as are disclosed in U.S. Patent Specification No. 4,158,667. Because of its greater purity, the compound is easier to handle and is relatively stable. This makes it a superior compound for formulating into pharmaceutical compositions.

Further, prostaglandin $I_2$, methyl ester is an intermediate for the preparation of the sodium salt of this methyl ester which is a well known and useful pharmacological

agent.

The substantially pure nature of the prostaglandin $I_2$, methyl ester of the present invention allows for the preparation of a purer form of the sodium salt than can be prepared from the known lower melting, less pure, form of prostaglandin $I_2$, methyl ester. Further, the high purity of the methyl ester of the present invention allows for the preparation of the sodium salt in a faster and less expensive way.

The best previously known method for preparing the sodium salt from

the low melting methyl ester is to treat it with sodium carbonate under carefully controlled conditions. A purer form of the sodium salt is prepared from the purer form of the methyl ester by reacting it with sodium hydroxide under much less controlled conditions. The problem with using the less pure form of the methyl ester is that it is difficult to measure the precise amount of starting material in order to determine the precise amount of hydrolyzing agent to be added. Thus, sodium carbonate is used to avoid excess base which would tend to decompose the product. However, the purer methyl ester may be measured more precisely and thus the exact amount of the sodium hydroxide may be used, thereby providing a simpler and less expensive means of obtaining a purer form of the sodium salt of prostaglandin $I_2$, methyl ester.

Prostaglandin $I_2$, methyl ester and its sodium salt are formulated into suitable pharmaceutical compositions in the manner given in U.S. Patent 4,158,667. Because of the improved purity of the compounds of the present invention, the pharmaceutical compositions therefrom represent substantially purer pharmaceutical compositions which cannot be obtained from the previously known compounds.

The less pure soft crystalline form of prostaglandin $I_2$, methyl ester is prepared according to the method of U.S. Patent 4,158,667 and Johnson <u>et al</u>., J. Am. Chem. Soc. 100:24 (1978). Surprisingly and unexpectedly, the prostaglandin $I_2$, methyl ester thus formed can be substantially purified to crystals having a higher melting point and greater degree of optical rotation than is disclosed in the prior art, by the method described herein.

The preparation of compounds of the present invention is illustrated in the following Examples.

Example 1 Preparation of substantially pure prostaglandin $I_2$, methyl ester.

A 5 l three-necked flask equipped with a mechanical stirrer, thermometer, and heating means is charged with 2085 ml of toluene and heated to 50° C. 700 ml of 1,5-diazobicyclo[5.4.0]undec-5-ene (DBU) are added. 407 g of 5-ξ-iodo-9-deoxy-6,9α-epoxy-$PGF_{1}\alpha$, methyl ester are added. After a few minutes, the temperature rises to 62° C and a precipitate begins to separate. The mixture is placed under nitrogen

and stirred at 45-50° C overnight. The mixture is assayed by TLC to determine the presence of starting material.

The mixture is cooled to 40° C and transferred to a wash tank where it is washed with four 2 l portions of water containing small amounts of sodium chloride. A small amount of triethylamine is added after each wash. The combined water washes are back extracted with 500 ml of toluene, and the combined organic material is dried over anhydrous magnesium sulfate. 10 g of activated charcoalare added and the mixture is filtered and divided into two equal portions. Each portion is concentrated using a rotary/vaporizing apparatus equipped with water aspirator at 38° C to yield two portions containing 164 and 149 g of an orange oil. The total time involved is less than two hrs for each portion.

Each concentrate is dissolved in 100 ml of ether containing a few ml of triethylamine. Hexane is added slowly to each portion until the mixture becomes cloudy and then 100 ml of ether is added. This procedure is continued until each solution contains about 500 ml of ether. The total combined volume of each of these portions is about 3 l.

If substantially pure prostaglandin $I_2$, methyl ester, is available from a previous systhesis the mixture is preferably seeded with these substantially pure crystals.

The mixtures are maintained at room temperature for 1 hr while a gelatinous precipitate separates. Hexane is added in small portions at room temperature, to a total volume of 3.7 to 4.0 l. The gelatinous precipitates are filtered.

Each precipitate is dissolved in 200-250 ml of ether containing 5-10 l of triethylamine. Each solution is then diluted with hexane to a slight cloudiness. Once again,if substantially pure prostaglandin $I_2$, methyl ester is available the mixture is seeded.

Each mixture is then diluted with hexane to a volume of 2 l. After the precipitates have separated they are collected on a filter. This recrystallization is repeated one or more times until the precipitate is obtained in the form of fine needles having a melting point above 50° C.

If the gel remains in the precipitate it is triturated with 1.5 l of 10% ether in hexane.

The combined weight of the two precipitates is 204 g.

The combined solids are dissolved in a mixture of 20 ml of triethylamine and 400 ml of ether by warming the mixture gently to 30° C. About 1 l of hexane is slowly added while the mixture is being stirred until the solution has a persistent cloudiness.

The mixture is warmed to about 41° C and stirred under a strong jet of nitrogen to evaporate the solvent and cool the mixture. If pure final product is available from a previous synthesis the solution is seeded at this point. Fine needles begin to separate. When crystallization of good needles is well underway, hexane is gradually added at 27-30° C for a total of 500 ml. Care should be taken not to overcool the mixture as this will cause gel formation.

The mixture is stirred at room temperature for 2 hr and then at 5° C for 2 hr so that the final temperature of the solution is 12° C.

The solid is collected on a filter and is dried under nitrogen and finally in a vacuum oven at 30° C overnight. The yield is 198.6 g (66%) of fine colorless needles having a melting point of 56.5 to 58.7° C. TLC analysis shows the product to be at least 97% pure having an optical rotation $[\alpha]_D$ of +102° (C = 1.033 g/100 ml of ethanol), and exhibiting IR peaks as shown in the subsequent Table.

The IR analysis was undertaken on a mineral oil mull in the range of 3800-600 wavenumbers ($cm^{-1}$). The spectrum was obtained using a Digilab Model 14D Fourier transform spectrophotometer. The peak tabulation in the Table was taken from a computer analysis of the spectrum using a Harris /7 computer. The spectrum is shown in the accompanying drawing.

Band Tabulation of the Infrared Spectrum of Substantially Pure $PGI_2$, Methyl Ester

| Band Frequency[1] | Intensity[2] | Band Frequency | Intensity |
|---|---|---|---|
| 3451 | 13 | 1108 | 66 |
| 3393 | 30, sh. | 1084 | 9 |
| 2922 | 0, M | 1069 | 15 |
| 2855 | 1, M | 1052 | 26 |
| 2724 | 83, M | 1041 | 27 |
| 2682 | 84, M | 1022 | 37 |
| 1794 | 90, sh. | 1008 | 58 |
| 1744 | 4 | 899 | 37 |
| 1711 | 7 | 973 | 2 |
| 1697 | 31 | 955 | 60 |
| 1687 | 35 | 910 | 38 |
| 1457 | 15, M | 892 | 59 |
| 1450 | 16, M | 857 | 49 |
| 1437 | 10 | 833 | 82 |
| 1417 | 33 | 827 | 76 |
| 1377 | 23, M | 814 | 72 |
| 1361 | 25 | 781 | 50 |
| 1352 | 32 | 755 | 47 |
| 1333 | 33 | 727 | 60 |
| 1316 | 13 | 721 | 59, M |
| 1286 | 26 | 707 | 57 |
| 1282 | 26 | 663 | 53 |
| 1265 | 52 | 613 | 45 |
| 1240 | 12 | | |
| 1228 | 43 | | |
| 1206 | 9 | | |
| 1175 | 17 | | |
| 1163 | 30 | | |
| 1133 | 26 | | |
| 1127 | 26 | | |

[1]Wavenumbers ($cm^{-1}$).
[2]Percent transmittance (%T), sh. = shoulder, M=possible interference from mineral oil.
Intensity at 3800 $cm^{-1}$ is 92.5%T.
Minimum intensity at 1919 $cm^{-1}$ is 96.9%T.

Example 2 Preparation of the substantially pure prostaglandin $I_2$, sodium salt

3.67 g (10.0 mmols) of the substantially pure prostaglandin $I_2$, methyl ester, prepared as above is dissolved in 20 ml of methanol and 10.0 ml (10.0 mmols) of sodium hydroxide is added using a volumetric pipette. The clear colorless solution is kept at room temperature overnight.

The bulk of the solution (having a pH of 7.0) is gradually diluted at room temperature with acetonitrile to slight cloudiness and swirled until the cloudiness is replaced with tiny crystals in a clear solution. This was repeated until the volume was 400 ml of a viscous crystal slurry. This slurry is chilled at 5° C for $1\frac{1}{2}$ hrs and the fine white crystals are collected, rinsed with acetonitrile and dried on filter paper under nitrogen atmosphere. The yield is 2.78 g (74.3%) of prostaglandin $I_2$, sodium salt. HPLC analysis shows that the titled product is 99.84% pure by weight. Carbon:hydrogen:sodium analysis is 64.44:8.47:6.38. The optical rotation $[\delta]_D$ in ethanol is + 95° (C = 1.0398); in DMF it is + 96° (C = 0.8815).

CLAIMS

1. A substantially pure crystalline form of prostaglandin $I_2$, methyl ester, characterized by the following physical properties: a melting point of from 54.7 to 60.6° C; $[\alpha]_D$ in ethanol of 96-104° C (C = 1.033); and exhibiting IR peaks (in a mineral oil mull) essentially at 3451, 3393, 2922, 2855, 2724, 2682, 1794, 1744, 1711, 1697, 1687, 1457, 1450, 1437, 1417, 1377, 1361, 1352, 1333, 1316, 1286, 1282, 1265, 1240, 1228, 1206, 1175, 1163, 1133, 1127, 1108, 1084, 1069, 1052, 1041, 1022, 1008, 988, 973, 955, 910, 892, 857, 833, 827, 814, 781, 755, 727, 721, 707, 663 and 613 wavenumbers ($cm^{-1}$).

2. A free-flowing substantially pure form of prostaglandin $I_2$, sodium salt, characterised in that it is greater than 99.0 percent pure by weight, using HPLC analysis.

3. A pharmaceutical composition which comprises prostaglandin $I_2$, sodium salt, which is greater than 99.0 pure by weight, using HPLC analysis, obtained from prostaglandin $I_2$, methyl ester;and an inert carrier.

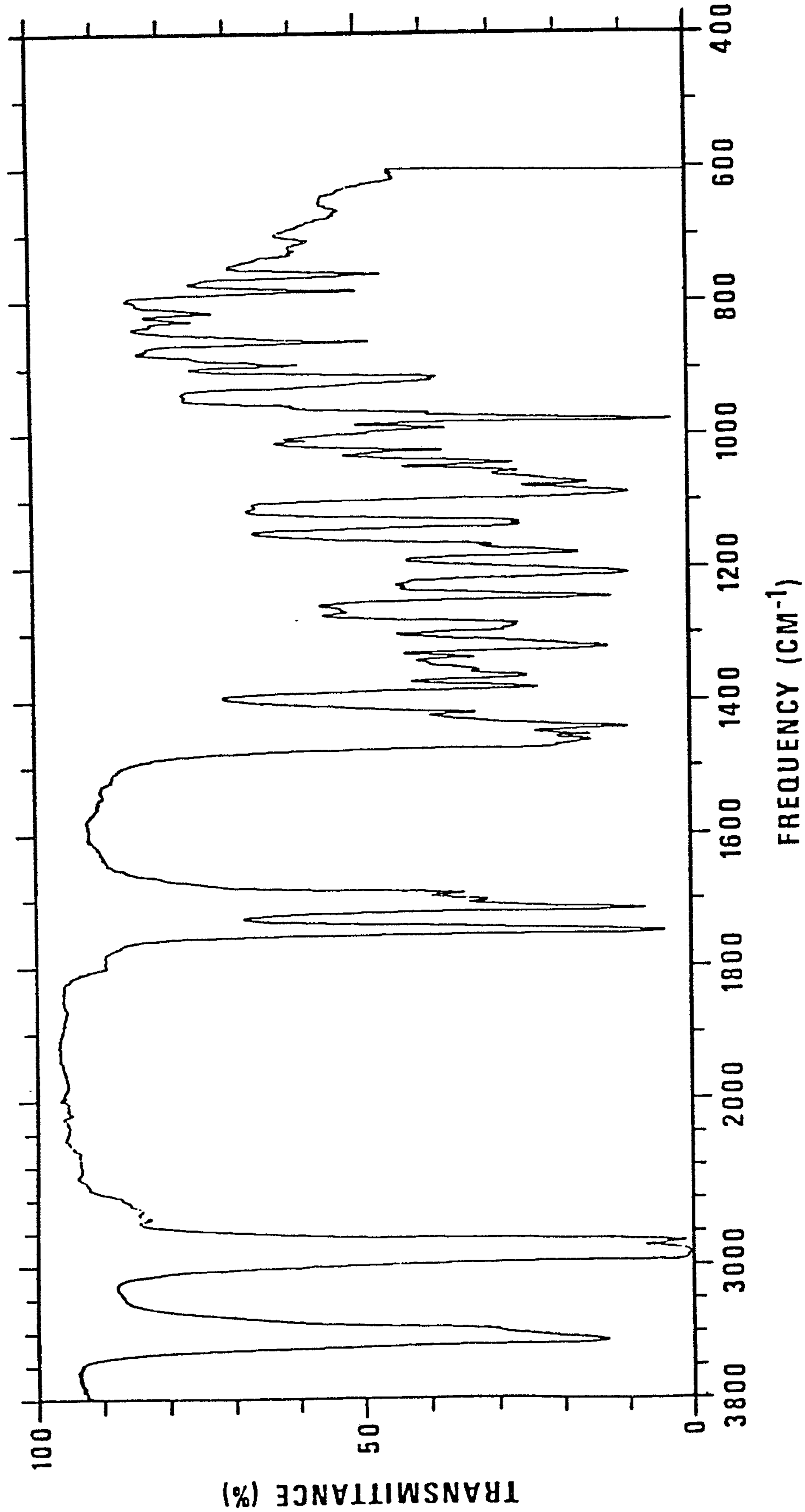

TRANSMITTANCE (%)
100
50
0
FREQUENCY (CM-1)
3800
3000
2000
1800
1600
1400
1200
1000
800
600
400